# EUROPÄISCHE PATENTSCHRIFT

(11) **EP 2 065 085 B1**
(45) Veröffentlichungstag und Bekanntmachung des Hinweises auf die Patenterteilung: **14.11.2012**
(21) Anmeldenummer: 08164951.9
(22) Anmeldetag: 24.09.2008
(51) Int. Cl.: B01F 7/00, B01F 13/08, B01F 15/00, C12M 1/00, C12M 1/02, F16C 32/04

(54) **Mischvorrichtung und Behälter für eine solche**
Mixing device and container for such
Dispositif de mélange et récipient le comportant

(30) Priorität: 30.11.2007 EP 07122018
(43) Veröffentlichungstag der Anmeldung: 03.06.2009
(73) Patentinhaber: Levitronix GmbH, 8005 Zürich (CH)
(72) Erfinder: Schöb, Reto, Dr., 9200 Gossau (CH)
(74) Vertreter: Sulzer Management AG

(56) Entgegenhaltungen:
- EP-A- 0 210 651
- EP-A- 1 618 905
- WO-A-2008/005611
- JP-A- 63 036 825
- US-A- 5 061 079
- US-A1- 2004 062 140
- US-A1- 2004 245 144

## Beschreibung

Die Erfindung betrifft eine Mischvorrichtung mit einem Behälter zum Mischen von Medien sowie einen Behälter für eine solche Mischvorrichtung.

In der pharmazeutischen und in der biotechnologischen Industrie werden häufig Lösungen und Suspensionen hergestellt, die eine sorgfältige Durchmischung der Komponenten verlangen. Beispiele hierfür sind die Deaktivierung von Viren durch massive Änderung des pH-Wertes oder die Herstellung von Puffern. Auch Prozesse in Bioreaktoren, in denen speziell herangezüchtete Mikroorganismen oder Zellen unter möglichst optimalen Bedingungen in einem Nährmedium kultiviert werden, um entweder die Zellen selbst, z.B. Gewebezellen, oder Teile von ihnen oder ihre Stoffwechselprodukte zu gewinnen, verlangen häufig eine sorgfältige Durchmischung der Zellkulturen mit Gasen.

Allen diesen Prozessen ist es gemeinsam, dass sie sehr häufig unter sterilen Bedingungen durchgeführt werden müssen, um eine Kontamination beispielsweise des gezüchteten Gewebes zu vermeiden. Üblicherweise hat man daher solche Prozesse in Edelstahl-, Glas- oder Chromtanks durchgeführt, die vorher aufwändig sterilisiert werden. In letzter Zeit zeichnet sich immer mehr der Trend ab, derartige Prozesse in flexiblen Kunststoffbeuteln durchzuführen, die für den Einmalgebrauch gedacht sind. Häufig werden solche Beutel bereits bei der Herstellung sterilisiert und dem Kunden in steriler Verpackung zugestellt. Beispiele für solche Bioreaktoren für den Einmalgebrauch finden sich in der WO-A-2005/118771 oder in der WO-A-2005/104708.

Für das Durchmischen der Komponenten sind Mischer oder Rührer in den Gefässen vorgesehen. Da die Prozesse häufig unter kontrollierter Atmosphäre durchgeführt werden, also in Gefässen, die gegenüber der Umgebung abgeschlossen sind, stellt die Durchführung der rotierenden Achsen in das Gefäss eine mögliche Quelle für eine Kontamination dar, beispielsweise auch durch Abrieb in den Gleitdichtungen der Welle. Ferner können sich in den Durchführungen Mikroorganismen ansammeln, welche beispielsweise die Sterilität gefährden. Diese Probleme lassen sich vermeiden bzw. lösen, indem man das Rühr- oder Mischelement magnetisch an einen ausserhalb des Gefässes befindlichen Drehantrieb koppelt. Eine derartige Lösung wird beispielsweise in der US-A-7,086,778 vorgeschlagen.

Aus der EP-A-1 618 905 ist eine Pump- oder Mischvorrichtung bekannt, bei welcher das in einem Gefäss für Flüssigkeiten vorgesehene Misch- oder Pumpelement mittels supraleitender Magnetegelagert wird.

Aus der WO-A-2008/005811 (Stand der tard der Technik unter Art. 54(3) EPÜ) ist eine Mischvorrichtung bekannt, bei welchem das Mischelement durch eine in einem Flüssigkeitstank bewegbare Führung schwenkbar ist.

Auch bei Verwendung von Rührern oder Mischern ist es häufig sehr schwierig, eine homogene und gradientenfreie Durchmischung der verschiedenen Komponenten zu gewährleisten. Durch die Mischer kommt es zu Strömungen mit unterschiedlichen Turbulenzen, wobei die Turbulenzen in der Regel in der Nähe der Mischer wesentlich stärker sind als in Bereichen, die weit entfernt von den Mischern sind. Dies hat zur Folge, dass sich eine homogene Durchmischung -wenn überhaupt - nur sehr langsam aufbaut. Hier will die Erfindung Abhilfe schaffen.

Es ist daher eine Aufgabe der Erfindung eine Mischvorrichtung sowie einen dafür geeigneten Behälter vorzuschlagen, der eine gute und homogene Durchmischung ermöglicht, insbesondere auch in Bioreaktoren.

Die diese Aufgabe lösenden Gegenstände der Erfindung sind durch die Merkmale der unabhängigen Patentansprüche gekennzeichnet.

Erfindungsgemäss wird also eine Mischvorrichtung vorgeschlagen mit einem Behälter zum Mischen von Medien, welcher mindestens einen Einlass für die zu mischenden Medien oder mindestens einen Auslass aufweist, wobei eine zentrale Ausnehmung vorgesehen ist, die sich in einer Längsrichtung in den Behälter hinein erstreckt und durch eine Wandung gegen den Innenraum des Behälters abgegrenzt ist, wobei in dem Behälter benachbart zu der Ausnehmung ein permanentmagnetisches Mischelement zum Mischen der Medien vorgesehen ist und wobei in der Ausnehmung eine Antriebseinheit vorgesehen ist, mit welcher das Mischelement Ober eine magnetische Kopplung in Rotation versetzt werden kann. Die Antriebseinheit ist in der Ausnehmung bewegbar angeordnet, sodass die Position des Mischelements in dem Behälter kontrolliert veränderbar ist. Die Wandung der Ausnehmung ist flexibel ausgestaltet und derart, dass die Ausdehnung der Ausnehmung, in der Längsrichtung veränderbar ist.

Bei dieser Mischvorrichtung kann das in dem Behälter befindliche Mischelement in einem weiten Bereich frei verschoben werden, wodurch eine deutlich bessere und räumlich homogenere Durchmischung erzielt wird. Da zudem die Positionierung des Mischelements ebenso wie sein Antrieb Ober eine magnetische Kopplung erfolgt, bedarf es keiner aufwändiger Einrichtungen im oder Durchführungen in den Behälter, sodass zum einen die Vorrichtung einfach vom Aufbau her ist und zum anderen die Gefahr unerwünschter Verunreinigungen im Behälter deutlich reduziert wird.

Dies kann beispielsweise realisiert werden, indem die Ausnehmung in Form oder nach dem Prinzip eines Balgs oder als flexibler Schlauch ausgestaltet ist. Dadurch wird eine Vielzahl zusätzlicher Ausgestaltungen und Varianten der Mischvorrichtung ermöglicht. Beispielsweise kann das Mischelement dann auch nach dem Prinzip eines Innenläufers magnetisch in der Antriebseinheit gelagert sein und von dieser angetrieben werden.

Eine bevorzugte Ausgestaltung besteht darin, dass die Antriebseinheit ein elektrischer Drehantrieb ist, der zum Antreiben des Mischelements magnetisch mit dem Mischelement gekoppelt ist. Das heisst, die Drehbewegung des Rotors eines elektrischen Drehantriebs wird über eine magnetische Kopplung zum Antreiben der Rotationsbewegung des Mischelements eingesetzt.

Eine andere bevorzugte Ausgestaltung besteht darin, dass die Antriebseinheit einen Stator umfasst, welcher zusammen mit dem Mischelement als Rotor einen elektrischen Drehantrieb bildet. Bei dieser Ausgestaltung ist also das Mischelement selbst Teil des elektrischen Drehantriebs, nämlich dessen Rotor. Besonders bevorzugt ist dabei das Mischelement als permanentmagnetischer Rotor ausgestaltet, der zusammen mit dem Stator einen lagerlosen Motor bildet, bei welchem der Stator als Lager- und Antriebsstator für den permanentmagnetischen Rotor ausgestaltet ist. Diese Ausgestaltung als lagerloser Motor ist äusserst kompakt und platzsparend, weil keine separaten Magnetlager für den Rotor benötigt werden, sondern der Stator sowohl das Antriebsmoment für den Rotor generiert als auch die magnetische Lagerung des Rotor realisiert.

Eine apparativ besonders einfach Ausgestaltung ist es, wenn die Antriebseinheit in der Ausnehmung hängend angeordnet ist. Dann kann die Antriebseinheit in einfacherweise über einen Seilzug oder eine Kette in der Ausnehmung auf und ab bewegt werden, um so die Position des Mischelements im Behälter kontroliert zu verändern.

Eine vorteilhafte Massnahme besteht darin, dass das innenliegende Ende der Ausnehmung eine becherartige Verstärkung, insbesondere aus Kunststoff aufweist. Diese Verstärkung dient als Auflagefläche für das Mischelement, wenn die Antriebseinheit deaktiviert ist. Die Verstärkung verhindert eine Verletzung der Wandung der Ausnehmung.

Eine vorteilhafte Variante besteht darin, dass die Antriebseinheit quer zur Längsrichtung der zentralen Ausnehmung bewegbar ist. Durch diese Massnahme lässt sich das Mischelement nicht nur in der Längsrichtung der Ausnehmung verschieben sondern auch in einer dazu senkrechten Ebene. Dies ist insbesondere bei grösseren Behältern vorteilhaft, um eine noch homogenere oder schnellere Durchmischung zu erreichen.

Besonders bevorzugt ist der Behälter als flexibler Beutel oder als sterilisierter flexibler Beutel ausgestaltet. Damit wird dem heute insbesondere für Bioreaktoren zu beobachtenden Trend Rechnung getragen, einfach und kostengünstig zu produzierende Behälter zu verwenden. Der Behälter ist vorzugsweise als Wegwerf-Behälter für den Einmalgebrauch ausgestaltet.

Dadurch entfallen aufwändige Reinigungs- und/oder Sterilisierungsvorgänge, wie sie üblicherweise in Edelstahl oder Glasbehältern anfallen.

Gemäss einer bevorzugten Ausgestaltung hat die Mischvorrichtung ein starres Stützgefäss, welches den Behälter aufnimmt, der vorzugsweise als flexibler Beutel ausgestalten ist. Diese Ausgestaltung ermöglicht es, den Behälter, der mit den Medien in Kontakt kommt, für den Einmalgebrauch auszulegen, während die anderen Komponenten mehrfach genutzt werden können.

Besonders bevorzugt ist die Mischvorrichtung als Bioreaktor oder als Fermenter ausgestaltet, weil hier die Vorteile der magnetischen Positionierbarkeit und der magnetischen Lagerung des Mischelements besonders gut genutzt werden können.

Ferner wird durch die Erfindung ein Behälter vorgeschlagen, für eine Mischvorrichtung, die erfindungsgemäss ausgestaltet ist, wobei der Behälter als flexibler Beutel für den Einmalgebrauch ausgestaltet ist, ein permanentmagnetisches Mischelement enthält und eine zentrale Ausnehmung aufweist, in welcher eine Antriebseinheit bewegbar angeordnet werden kann, wobei die Ausnehmung durch eine Wandung begrenzt wird, die flexibel ist und so ausgestaltet ist, dass die Ausdehnung der Ausnehmung in einer Längsrichtung veränderbar ist

Die Ausgestaltung des Beutels mit der zentralen Ausnehmung ermöglicht es, die Position des Mischelements in dem Behälter kontrolliert zu verändern.

Weiter vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher ertäutert. In der schematischen Zeichnung zeigen teilweise im Schnitt:
- Fig. 1:: eine schematische Darstellung eines ersten Ausführungsbeispiels einer erfindungsgemässen Mischvorrichtung,
- Fig.2:: eine Schnittdarstellung der Antriebseinheit und des Mischelements entlang der Schnittlinie II-II in Fig. 3,
- Fig. 3:: eine Schnittdarstellung entlang der Schnittlinie III-III in Fig. 2,
- Fig. 4:: ein Variante des ersten Ausführungsbeispiels in einer Schnittdarstellung
- Fig. 5:: eine Variante für die Antriebseinheit und das Mischelement in einer Schnittdarstellung entlang der Schnittlinie V-V in Fig. 6,
- Fig. 6:: eine Schnittdarstellung entlang der Schnittlinie VI-VI in Fig. 5,
- Fig. 7:: eine Schnittdarstellung der Antriebseinheit und des Mischelements eines zweiten Ausführungsbeispiels der erfindungsgemässen Mischvorrichtung entlang der Schnittlinie VII-VII in Fig. 8,
- Fig. 8:: eine Schnittdarstellung entlang der Schnittlinie VIII-VIII in Fig. 7,
- Fig. 9:: eine Schnittdarstellung entlang der Schnittlinie IX-IX in Fig. 7, und
- Fig. 10:: eine schematische Darstellung eines dritten Ausführungsbeispiels einer erfindungsgemässen Mischvorrichtung.

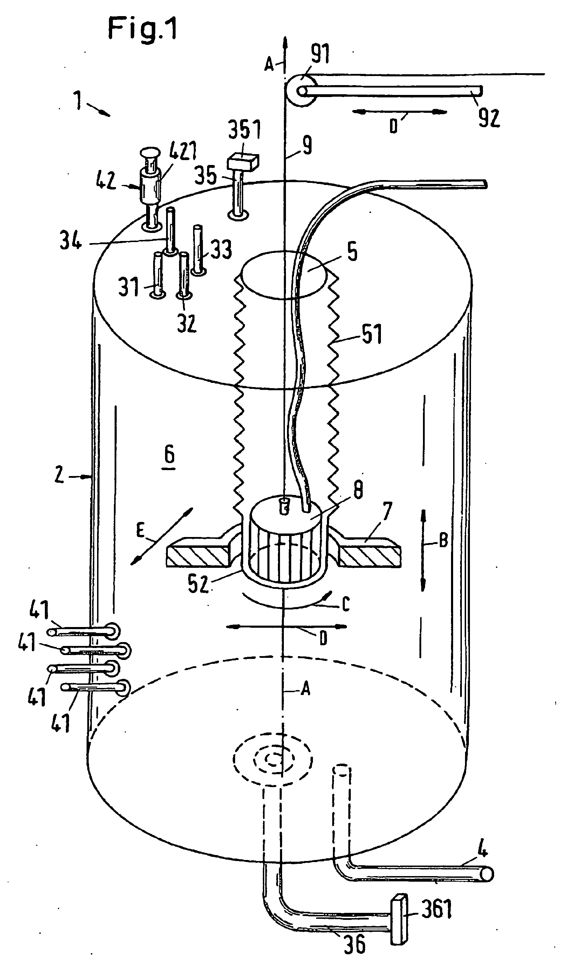

Fig. 1 zeigt in einer schematischen, teilweise geschnittenen Darstellung ein erstes Ausführungsbeispiel einer erfindungsgemässen Mischvorrichtung, die gesamthaft mit dem Bezugszeichen 1 bezeichnet ist. Bei der folgenden Beschreibung der Erfindung wird mit beispielhaftem Charakter auf den für die Praxis wichtigen Fall Bezug genommen, dass die erfindungsgemässe Mischvorrichtung als Bioreaktor oder als Fermenter ausgestaltet ist. Es versteht sich jedoch, dass die Erfindung nicht auf solche Ausgestaltungen beschränkt ist, sondern ganz allgemein Mischvorrichtungen betrifft, mit denen Medien gemischt werden. Insbesondere können diese Medien Fluide oder Feststoffe, vorzugsweise Pulver, sein. Die erfindungsgemässe Mischvorrichtung eignet sich zum Mischen von Flüssigkeiten untereinander und/oder zum Mischen von mindestens einer Flüssigkeit mit einem Pulver oder sonstigen Feststoff und/oder zum Mischen von Gasen mit Flüssigkeiten und/oder Feststoffen.

Das in Fig. 1 dargestellte erste Ausführungsbeispiel zeigt eine als Bioreaktor ausgestaltete Mischvorrichtung 1, die einen Behälter 2 zum Mischen von Medien umfasst. Dieser Behälter 2 kann als flexibler Beutel, beispielsweise als Plastik- oder Kunststoffsack ausgestaltet sein oder auch als starrer Behälter 2, beispielsweise aus Edelstahl, Glas, Chromstahl oder Kunststoff. Im Hinblick auf die neueren Entwicklungen sind jedoch flexible Ausgestaltungen des Behälters 2 bevorzugt.

Der Behälter 2 umfasst mindestens einen Einlass, im vorliegenden Beispiel sechs Einlässe 31,32,33,34,35,36 für die zu mischenden Medien, sowie mindestens einen Auslass 4. Zudem sind in der Wandung des Behälters 2 mehrere Anschlüsse 41 (Ports) vorgesehen. Durch diese können beispielsweise Messsensoren eingeführt werden, um die Temperatur, den pH-Wert oder auch andere Grössen messtechnisch zu bestimmen. Die Anschlüsse 41 können ferner dazu genutzt werden, Proben aus dem Behälter 2 zu entnehmen.

Es ist auch möglich, dass der Behälter 2 nur einen Einlass oder nur einen Auslass aufweist. Auch sind solche Ausgestaltungen möglich, die nur einen Einlass und keinen Auslass oder nur einen Auslass und keinen Einlass aufweisen. Bei derartigen Ausgestaltungen dient dann die nur eine Öffnung sowohl als Einlass als auch als Auslass.

Es ist eine zentrale Ausnehmung 5 vorgesehen, welche sich in einer Längsrichtung A in den Behälter 2 hinein erstreckt und welche durch eine Wandung 51 gegen den Innenraum 6 des Behälters 2 abgegrenzt ist. Im vorliegenden Beispiel ist der Behälter 2 im wesentlichen zylindrisch ausgestaltet, die Längsrichtung A ist die Richtung der Zylinderachse. Die Ausnehmung 5 erstreckt sich von der darstellungsgemäss oberen Stirnflache des zylindrischen Behälters 2 in dessen Innenraum 6 und ist durch die Wandung 51 dichtend von dem Innenraum 6 abgegrenzt, sodass kein Medium aus dem Innenraum 6 des Behälters 2 in die Ausnehmung 5 eindringen kann.

Die Ausnehmung 5 ist mit einer flexibel ausgestalteten Wandung 51 versehen derart, dass die Ausdehnung der Ausnehmung 5 in der Längsrichtung A veränderbar ist. Im vorliegenden Ausführungsbeispiel ist die Längsrichtung A veränderbar ist. Im vorliegenden Ausfühnjngsbeispiet ist die Wandung 51 als Balg ausgestaltet, der in Längsrichtung A zusammenschiebbar und auseinanderziehbar ist - vergleichbar mit einer Ziehharmonika.

Alternativ kann die flexible Ausgestaltung der Wandung 51 auch realisiert werden, indem ein Schlauch aus flexiblem Gummi oder ein Schlauch aus einem flexiblen Kunststoff verwendet werden.

Im Innenraum 6 des Behälters 2 ist benachbart zu der Ausnehmung 5 ein permanentmagnetisches Mischelement 7 vorgesehen, mit welchem die Medien durchmischt werden können. Ferner ist in der Ausnehmung 5 eine Antriebseinheit 8 vorgesehen, mit welcher das Mischelement 7 Ober eine magnetische Kopplung in Rotation versetzt werden kann, wie dies der Pfeil C in Fig. 1 andeutet.

Erfindungsgemäss ist die Antriebseinheit 8 in der Ausnehmung 5 bewegbar angeordnet, sodass die Position des Mischelements 7 in dem Behälter 2 kontrolliert veränderbar ist.

Dazu ist bei diesem Ausführungsbeispiel die Antriebseinheit 8 an einer Schnur 9 oder einem Seil oder einem Zug in der Ausnehmung 5 hängend angeordnet. Die Schnur 9 verläuft über eine Umlenkrolle 91 zu einer in Fig. 1 nicht dargestellten Wickelvorrichtung, beispielsweise einer Winde, mit der die Schnur 9 motorisch oder händisch auf- und abgewickelt werden kann. Durch diese Massnahme ist die Antriebseinheit 8 in der Ausnehmung 5 kontrolliert bewegbar - und zwar darstellungsgemäss aufwärts und abwärts entlang der Längsrichtung A. Im Betriebszustand folgt das Mischelement 7 durch die magnetische Kopplung der Antriebseinheit 8 und kann auf diese Weise kontrolliert entlang der Längsrichtung A auf- und ab bewegt werden.

Eine bevorzugte Massnahme besteht darin, dass das innenliegende Ende der Ausnehmung 5 - also gemäss der Darstellung in Fig. 1 das untere Ende der Ausnehmung 5 - eine becherartige Verstärkung 52 zur Aufnahme der Antriebseinheit 8 aufweist. Die Antriebseinheit 8 liegt dann auf dem Boden der becherartigen Verstärkung 52 auf.

Die Ausnehmung 5 bzw. die Wandung 51 ist so ausgestaltet, dass sie sich im Wesentlichen über die gesamte Erstreckung des Behälters 2 in Längsrichtung A ausdehnen bzw. zusammenschieben lässt. Dazu werden vorzugsweise die elastischen Eigenschaften der Wandung 51 so gewählt, dass die Ausnehmung 5 durch die Gewichtskraft der Antriebseinheit 8 bis zum darstellungsgemäss unteren Ende, also dem Boden des Behälters 2 dehnbar ist. Bei einer Aufwärtsbewegung der Antriebseinheit 8 zieht sich die Wandung 51 durch ihre elastischen Eigenschaften zusammen, sodass sich die Ausnehmung 5 bezüglich der Längsrichtung A verkürzt. Vorzugsweise verbleibt dabei die Antriebseinheit 8 in der becherartigen Verstärkung 52.

Während des Betriebs der Mischvorrichtung 1 kann nun die Antriebseinheit 8 durch Betätigung der Wickelvorrichtung in der Ausnehmung 5 auf- und abbewegt werden wie dies der Doppelpfeil B in Fig. 1 andeutet. Dadurch erfolgt ein Ausdehnen oder ein Zusammenziehen der Wandung 51 in Längsrichtung A. Aufgrund der magnetischen Kopplung folgt das Mischelement 7 der Bewegung der Antriebseinheit 8 in Längsrichtung A und kann auf diese Weise in dem Behälter 2 in jede beliebigen Position bezüglich der Längsrichtung A gebracht werden und dort gehalten werden. Somit ist das Mischelement bezüglich der Längsrichtung A zwischen dem darstellungsgemäss unteren und oberen Ende des Behälters 2 in Fig. 1 bewegbar.

Je nach Anwendungsfall ist es im Hinblick auf eiune besonders gute Durchmischung besonders vorteilhaft, wenn das Mischelement 7 zyklisch in dem Behälter 2 auf und ab bewegt wird.

Die Antriebseinheit 8 versetzt das Mischelement 7 im Betriebszustand über die magnetische Kopplung in Rotation, wie dies der Pfeil C in Fig. 1 andeutet, um so eine gute Durchmischung der Medien zu gewährleisten.

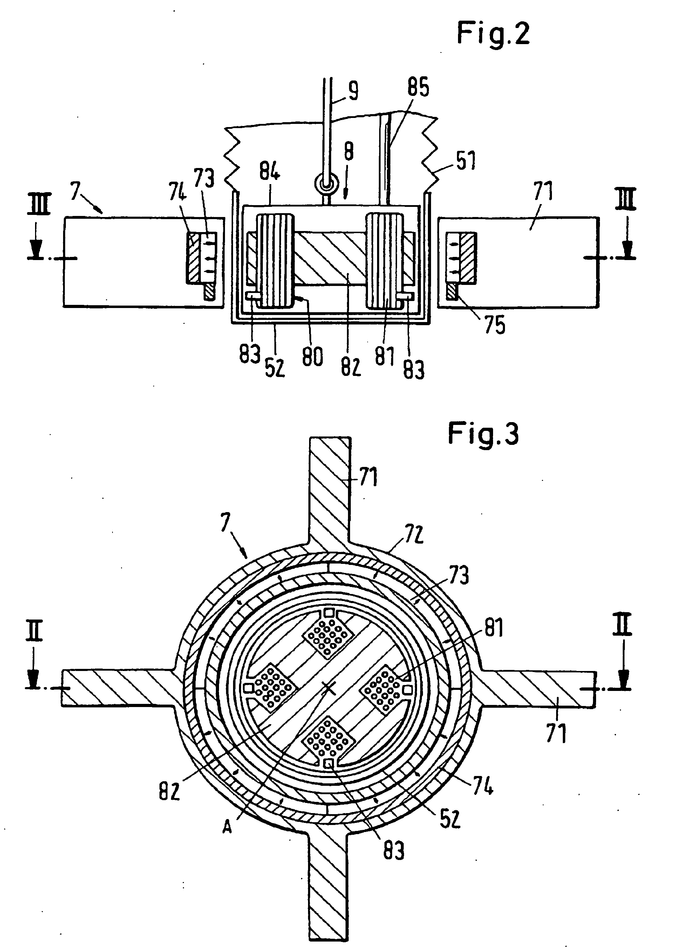

Im Folgenden wird der Antrieb und die Lagerung des Mischelements 7 des ersten Ausführungsbeispiels näher erläutert. Dazu ist in Fig. 2 die Antriebseinheit 8 und das Mischelement 7 im Schnitt dargestellt, wobei der Schnitt entlang der Schnittlinie II-II in Fig. 3 erfolgte. Fig. 3 zeigt die Antriebseinheit 8 und das Mischelement 7 in einer zu Fig. 2 senkrechten Schnittdarstellung entlang der Schnittlinie III-III in Fig. 2.

Bei dem ersten Ausführungsbeispiel umfasst die Antriebseinheit 8 einen Stator 80, der zusammen mit dem permanentmagnetischen Mischelement 7 einen elektrischen Drehantrieb bildet. Das Mischelement 7 ist hier also der Rotor eines elektromagnetischen Drehantriebs. Besonders bevorzugt ist das Mischelement 7 als permanentmagnetischer Rotor ausgestaltet, der zusammen mit dem Stator 80 einen lagerlosen Motor bildet, bei welchem der Stator als Lager- und Antriebsstator für den permanentmagnetischen Rotor ausgestaltet ist.

Ein solcher lagerloser Motor wird z. B. in der EP-A-0 819 330 bzw. der US-A-6,100,618 offenbart. Mit dem Begriff lagerloser Motor ist gemeint, dass das Mischelement 7 vollkommen magnetisch gelagert ist, wobei keine separaten Magnetlager vorgesehen sind. Der Stator 80 ist dazu als Lager- und Antriebsstator ausgestaltet, er ist also sowohl Stator des elektrischen Antriebs als auch Stator der magnetischen Lagerung. Dazu umfasst die Statorwicklung 81 eine Antriebswicklung der Polpaarzahl p sowie eine Steuerwicklung der Polpaarzahl p±1. Mit diesen beiden Wicklungen lässt sich ein magnetisches Drehfeld erzeugen, welches zum einen ein Drehmoment auf das Mischelement 7 ausübt, das dessen Rotation bewirkt, und welches zum anderen eine beliebig einstellbare Querkraft auf das Mischelement ausübt, sodass dessen radiale Position aktiv steuerbar bzw. regelbar ist. Somit sind drei Freiheitsgrade des Mischelements aktiv regelbar. Bezüglich dreier weiterer Freiheitsgrade, nämlich seiner axialen Auslenkung in Richtung der Drehachse und Verkippungen bezüglich der zur Drehachse senkrechten Ebene (zwei Freiheitsgrade) ist das Mischelement 7 passiv magnetisch, das heisst nicht ansteuerbar, durch Reluktanzkräfte stabilisiert. Bezüglich weiterer Details eines solchen lagerlosen Motors sei hier auf die bereits zitierten Dokumente verwiesen.

Der Stator 80 umfasst neben der Statorwicklung 81 ein Statorblechpaket 82 welches die Statorwicklung 81 trägt. Ferner sind mehrere Sensoren 83 vorgesehen, mit welchen beispielsweise die Position des Mischelements 7 bezüglich des Stators 80 bestimmbar ist oder die Lage der Rotormagnetisierung oder andere Messgrössen, die für den Betrieb des lagerlosen Motors notwendig sind bzw. Verwendung finden.

Der Stator 80 ist in einem Gehäuse 84 angeordnet, wobei eine eingekapselte (gepottete) Ausführung möglich ist, um den Stator 80 vor Fluiden zu schützen. Der Stator 80 ist über Versorgungsleitungen 85 mit einer nicht dargestellten Steuer- und Versorgungseinheit verbunden, welche den Stator 80 mit Energie versorgt und die Regeleinrichtungen für den Antrieb und die magnetische Lagerung des Mischelements 7 umfasst.

Bezüglich der in Fig. 2 und Fig. 3 dargestellten Ausgestaltung des elektromagnetischen Drehantriebs wird auf die EP-A-0 990 297 und die EP-A-0 990 296 verwiesen.

Das Mischelement 7 umfasst einen Grundkörper 72, der im wesentlichen ringförmig mit mehreren, sich in radialer Richtung erstreckenden Flügeln 71 ausgestaltet ist. Der Grundkörper besteht üblicherweise aus einem Kunststoff oder aus Chromstahl, der sterilisierbar ist. In den Grundkörper eingelagert ist ein ringförmiger Permanentmagnet 73, welcher aus mehreren permanentmagnetischen Segmenten aufgebaut sein kann, sowie ein ebenfalls ringförmiger magnetischer Rückschluss 74, vorzugsweise ein Eisenrückschluss, welcher den Permanentmagneten 73 radial aussen liegend umschliesst. Ferner ist darstellungsgemäss unterhalb des Permanentmagneten 74 ein Sensorring 75, beispielsweise aus Aluminium, vorgesehen, welcher Sensoren enthalten kann und/oder mit den Sensoren 83 im Stator 80 zusammenwirkt. Der ringförmige Permanentmagnet 73 ist bei diesem Ausführungsbeispiel als vierpoliger Magnet ausgestaltet (Polparzahl 2) und umfasst vier Ringsegmente, die jeweils homogen in radialer Richtung magnetisiert sind, wobei benachbarte Ringsegmente jeweils in die entgegengesetzte Richtung magnetiesiert sind, wie dies durch die Pfeile ohne Bezugszeichen angedeutet ist. Hieraus resultiert ein vierpoliger blockförmig oder rechteckförmig magnetisierter Rotor bzw. ein derartig magnetisiertes Mischelement 7.

Natürlich sind auch viele andere Arten der Magnetisierung des Mischelements 7 möglich.

Bei dem in den Fig. 1,2,3 dargestellten Ausführungsbeispiel ist noch eine weitere vorteilhafte Massnahme realisiert, nämlich, dass die Antriebseinheit 8 quer zur Längsrichtung A der zentralen Ausnehmung 5 bewegbar ist.

Dazu ist die Umlenkrolle 91 an einer Stange 92 (Fig. 1) befestigt. Die Stange 92 ist mit einem nicht dargestellten Antrieb versehen, mit welchem die Stange 92 - und damit die Umlenkrolle 91 - senkrecht zur Längsrichtung A hin- und her bewegbar ist. Gemäss der Darstellung in Fig. 1 ist die Umlenkrolle 91 nach links und nach rechts bewegbar, wie dies der Doppelpfeil mit dem Bezugszeichen D andeutet. Diese Bewegung überträgt sich auf die Antriebseinheit 8 und damit auf das magnetisch gekoppelte Mischelement 7, sodass das Mischelement 7 durch die Kombination der lateralen Bewegung der Umlenkrolle 91 mit der Auf- und Abbewegung in zwei Dimensionen bewegt und positioniert werden kann. Aufgrund der flexiblen Ausgestaltung der Wandung 51 kann diese auch der zweidimensionalen Bewegung der Antriebseinheit 8 folgen.

Es versteht sich, dass natürlich auch solche Ausgestaltungen möglich sind, bei denen die Antriebseinheit 8 und damit das Mischelement 7 in allen drei Dimensionen innerhalb des Behälters 2 bewegt werden können. Eine weitere Möglichkeit besteht darin, alternativ oder ergänzend die Stange 92 schwenkbar zu lagern beispielsweise derart, dass sie um eine zur Längsrichtung A parallele Achse drehbar bzw. schwenkbar ist.

Im Betrieb funktioniert die Mischvorrichtung 1, die hier als Bioreaktor ausgestaltet ist, wie folgt.

Insbesondere bei der Ausgestaltung des Behälters 2 für den Einmalgebrauch ist dieser als flexibler Beutel, vorzugsweise als Plastikbeutel ausgestaltet. Auch das Mischelement 7 ist dann üblicherweise als Wegwerf-Teil für den Einmalbetrieb konzipiert und bereits in den Behälter 2 integriert. In der Ausnehmung 5 kann dann ein nicht dargestelltes Eisenteil vorgesehen sein, welches das Mischelement 7 in der Nachbarschaft der Ausnehmung 5 hält, solange sich die Antriebseinheit 8 noch nicht in der Ausnehmung 5 befindet. Der Behälter 2 wird aufgestellt und das nicht dargestellte Eisenteil aus der Ausnehmung 5 entfernt. Die Antriebseinheit 8 wird in die Ausnehmung 5 eingeführt. Der Behälter 2 wird mit den zu mischenden Medien befüllt. Für die Flüssigkeiten und eventuell beizufügende Feststoffe, die beispielsweise in Pulverform vorliegen, sind die Einlässe 31, 32, 33, 34 vorgesehen. Die Flüssigkeit ist beispielsweise eine Nährlösung, z.B. eine Zuckerlösung. Für die Zuführung der gasförmigen Komponenten sind zwei Gaszuführungen 35 und 36 vorgesehen, von denen jede mit einem Filterelement 351 bzw. 361 versehen ist. Die Gaszuführung 35 dient dazu, ein Gas von oben auf die im Behälter 2 befindliche Flüssigkeit aufzubringen. Die Gaszuführung 36 dient dazu, ein Gas von unten in die Flüssigkeit im Behälter 2 einzublasen (Sparge).

Nachdem der Behälter 2 mit den zu mischenden Medien befüllt ist, wird mittels der Antriebseinheit 8 das magnetisch gelagerte Mischelement 7 in Rotation versetzt (Pfeil C) und durchmischt die Medien im Behälter 2. Dabei kann das Mischelement 7 durch eine entsprechende Bewegung der Antriebseinheit 8 in beliebiger Weise entlang der Längsrichtung A auf- und ab bewegt werden (gemäss der Darstellung in Fig. 1) und durch eine entsprechende Bewegung der Umlenkrolle 91 darstellungsgemäss nach rechts und nach links. Durch die Bewegung des Mischelements 7 ist eine homogene, effiziente Durchmischung erzielbar.

Für viele Anwendungen ist es ausreichend, das Mischelement 7 auf- und ab zu bewegen. Bei lateral sehr gross ausgedehnten Behältern 2 kann es vorteilhaft sein, das Mischelement zusätzlich quer zur Längsrichtung A in mindestens einer Richtung hin- und her zu bewegen. Diese Hin- und Herbewegung kann gemäss der Darstellung in Fig. 1 nach rechts und links erfolgen, wie dies der Doppelpfeil D in Fig. 1 andeutet oder nach vorne und hinten wie dies der Doppelpfeil E in Fig. 1 andeutet. Natürlich sind auch krummlinige Bewegungen quer zur Längsrichtung A möglich, beispielsweise durch ein Schwenken oder Drehen der Stange A um eine zur Längsachse A parallele Achse.

Besonders vorteilhaft ist es, wenn das Mischelement 7 über die gesamte Erstreckung des Behälters 2 in Längsrichtung A bewegbar und positionierbar ist, also darstellungsgemäss vom Boden des Behälters 2 bis zu seinem oberen Ende.

An dem Behälter 2 ist ferner ein Gasauslass 42 vorgesehen, durch welchen ein Gas aus dem Behälter 2 entweichen kann. Der Gasauslass 42 ist mit einem Filter 421 versehen.

Während des Betriebs können durch die Anschlüsse 41 Proben aus dem Innenraum 6 des Behälters 2 entnommen werden. Ferner können Messsensoren in den Innenraum 6 eingeführt werden, um Prozessparameter wie beispielsweise die Temperatur, zu überwachen.

Der Behälter 2 kann über den Auslass 4 geleert werden.

Bei diesem ersten Ausführungsbeispiel umfasst die Antriebseinheit 8 einen Stator 80, der zusammen mit dem permanentmagnetischen Mischelement 7 einen elektrischen Drehantrieb bildet. Dieser ist vorzugsweise als lagerloser Motor ausgestaltet. Natürlich sind aber auch andere Ausgestaltungen des elektrischen Drehantriebs möglich, beispielsweise Ausgestaltungen mit separaten Magnetlagern für das Mischelement, oder die Kombination von magnetischen Lagern mit anderen Lagern.

Das in den Fig. 1,2,3 dargestelte Ausführungsbeispiel umfasst einen elektrischen Drehantrieb, der nach dem Prinzip des Aussenläufers aufgebaut ist, d. h. der magnetisch wirksame Teil des Mischelements 7 befindet sich ausserhalb des Stators 80.

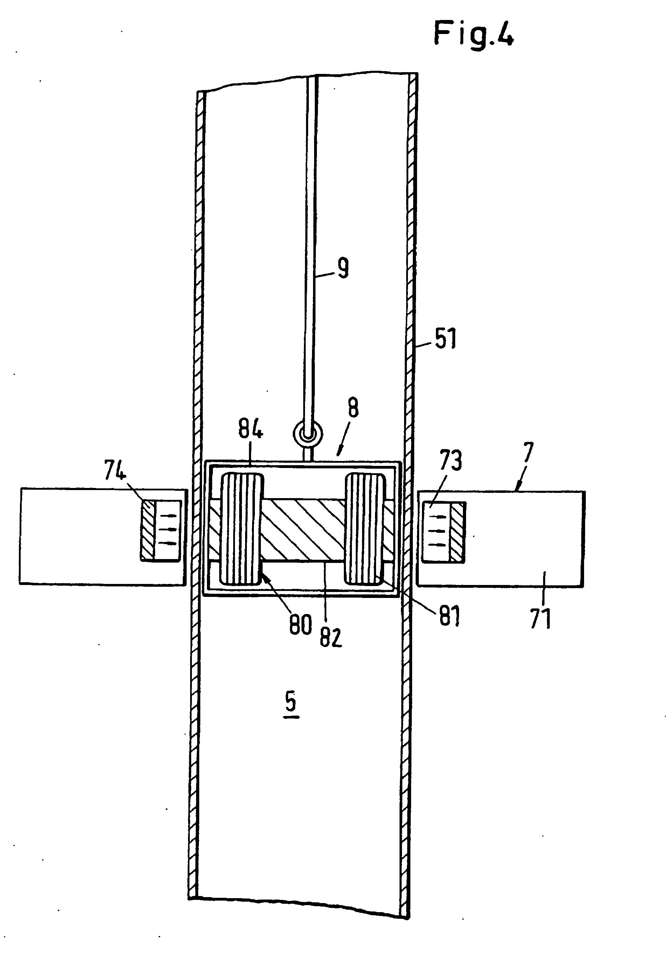

Insbesondere bei solchen Ausführungsbeispielen, die nach dem Prinzip des Aussenläufers aufgebaut sind, ist es auch möglich, die Wandung 51 der Ausnehmung 5 bzw. die Ausnehmung 5 nicht flexibel auszugestalten, sondern beispielsweise als starres Rohr, innerhalb dessen die Antriebseinheit 8 und insbesondere der Stator 80 entlang der Längsrichtung A bewegbar ist, also in einer der Fig. 1 entsprechenden Darstellung auf- und abwärts bewegbar ist. Eine solche Variante des ersten Ausführungsbeispiels ist in Fig. 4 in einer Schnittdarstellung gezeigt. Hier ist die Wandung 51 der Ausnehmung 5 als starres Rohr ausgebildet, dessen Längserstreckung sich im Betrieb nicht ändert. Auch bei dieser Ausführungsform erstreckt sich die Ausnehmung 5 bzw. die starre Wandung 51 vorzugsweise im Wesentlichen über die gesamte Ausdehnung des Behälters 2 in Richtung der Längsachse
A. Die bisher gemachten Erläuterungen gelten für die Variante in Fig. 4 in sinngemäss gleicher Weise. Auf die Darstellung der Sensorik und der Versorgunbgsleitungen ist in Fig. 4 verzichtet worden.

Da das Mischelement 7 aussen um die Ausnehmung 5 herum angeordnet ist, kann es auch bei einer starren Ausgestaltung der Wandung 51 der Bewegung des Stators 80 in der Längsrichtung A folgen.

Eine andere Variante des in den Fig. 1,2,3 dargestellten Ausführungsbeispiels besteht darin, den elektrischen Drehantrieb als Innenläufer auszugestalten.

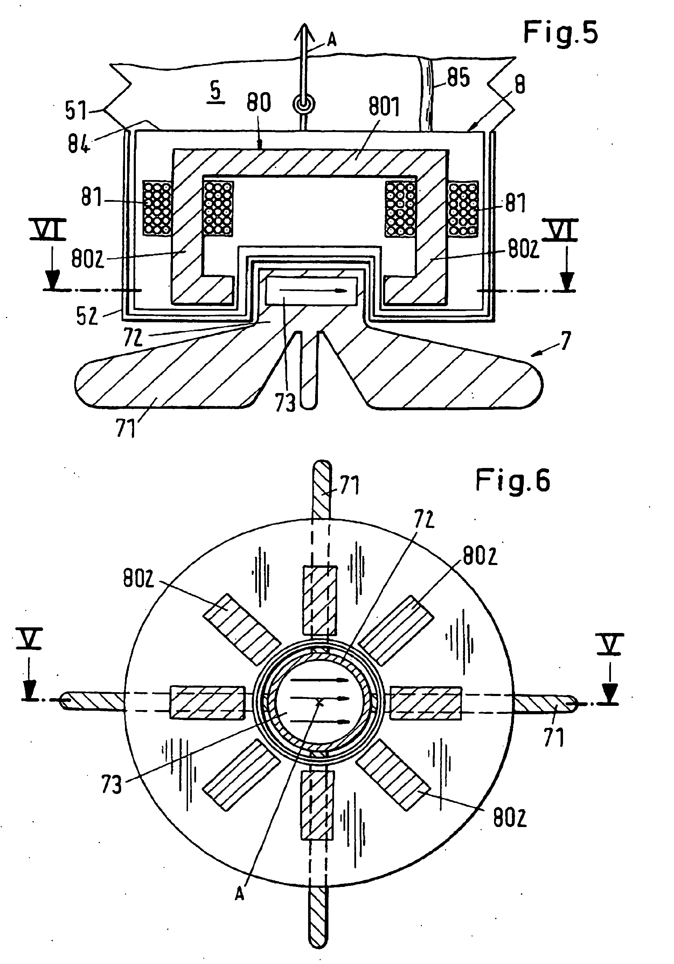

Die Fig. 5 und 6 zeigen eine solche Variante des ersten Ausführungsbeispiels. Fig. 5 zeigt die Antriebseinheit 8 und das Mischelement 7 in einer zu Fig. 3 analgen Schnittdarstellung. Die Schnittlinie V-V ist in Fig. 6 eingezeichnet. Fig. 6 zeigt die Antriebseinheit 8 und das Mischelement 7 in einer zu Fig. 5 senkrechten Schnittdarstellung entlang der Schnittlinie VI-VI in Fig. 5.

Im Folgenden wird nur auf die Unterschiede zu der Ausgestaltung gemäss den Fig. 1-3 eingegangen. Ansonsten gelten die vorangehenden Erläuterungen in sinngemäss gleicher Weise auch für die in Fig. 5 und 6 gezeigte Variante.

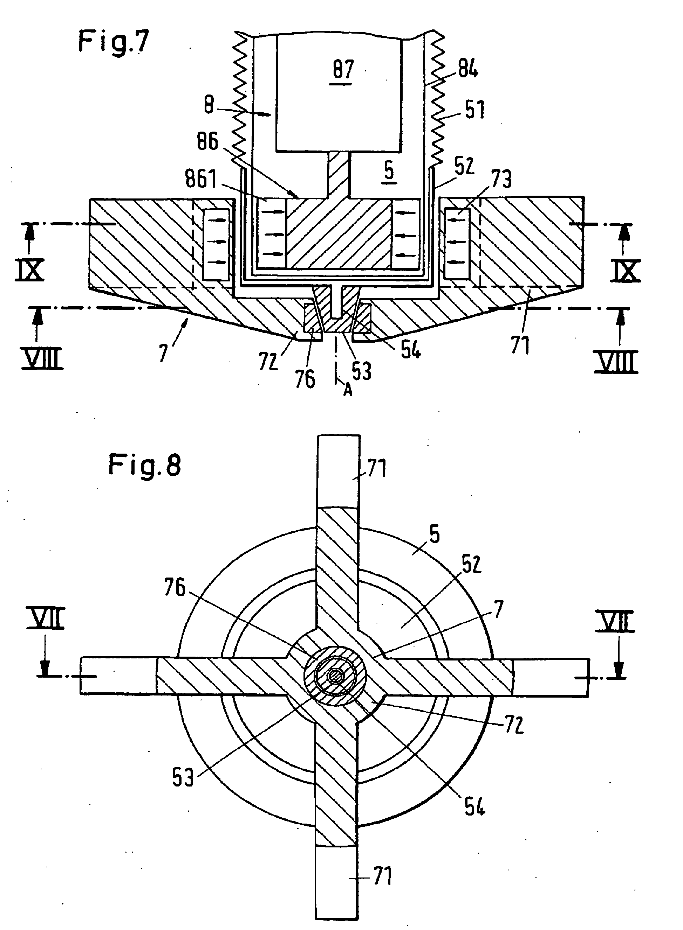

Auch bei dieser Variante ist der elektrische Drehantrieb als lagerloser Motor ausgestaltet, mit dem Lager- und Antriebsstator 80 und dem als Rotor dienenden Mischelement 7. Der Stator 80 (siehe insbesondere Fig. 5) ist bei dieser Variante nach dem Prinzip des Tempelmotors aufgebaut. Bei einem Tempelmotor, wie er beispielsweise in der WO-A-98/11650 offenbart ist (siehe dort Fig. 8k und zugehörige Textpassagen) weist der Stator 80 mehrere durch einen Rückschluss 801 verbundene Statorzähne 802 auf, die jeweils L-förmig ausgebildet sind, wobei sich der längere Schenkel in der Längsrichtung erstreckt, und der kürzere Schenkel radial nach innen verläuft. Die Statorwicklungen 81 sind jeweils auf den längeren Schenkeln der Statorzähne 802 vorgesehen. Der magnetisch wirksame Teil des als Rotor dienenden Mischelements 7 ist zwischen den kürzeren Schenkeln der Statorzähne 802 gelagert.

Das als Rotor dienende Mischelement 7 umfasst den Grundkörper 72, der mit mehreren, sich in radialer Richtung erstreckenden Flügeln 71 ausgestaltet ist. In den Grundkörper eingelagert ist ein scheibenförmiger Permanentmagnet 73, dessen Magnetisierung in den Fig. 5 und 6 durch die Pfeile ohne Bezugszeichen angedeutet ist. Der Permanentmagnet 73 ist zweipolig (Polpaarzahl 1) sinusförmig magnetisiert, wobei natürlich viele andere Magnetisierungsformen möglich sind. Der Permanentmagnet ist gemäss der Darstellung in Fig. 5 oberhalb der Flügel 71 in einem zentralen Teil des Grundkörpers 72 vorgesehen, der im Betriebszustand zwischen den kürzeren Schenkeln der Statorzähne 802 gelagert ist. Dementsprechend befinden sich die Flügel darstellungsgemäss unterhalb des Stators 80.

In den Fig. 7,8 und 9 ist die Antriebseinheit 8 und das Mischelement 7 eines zweiten Ausführungsbeispiels der erfindungsgemässen Mischvorrichtung 1 dargestellt. Im Folgenden wird nur auf die Unterschiede zu dem ersten Ausführungsbeispiel eingegangen. Die im Zusammenhang mit dem ersten Ausführungsbeispiel gemachten Erläuterungen gelten in sinngemäss gleicher Weise auch für das zweite Ausführungsbeispiel. Insbesondere sind gleiche oder von der Funktion her gleichwertige Teile mit den gleichen Bezugszeichen bezeichnet.

Bei dem zweiten Ausführungsbeispiel umfasst die Antriebseinheit 8 einen elektrischen Drehantrieb 87, der zum Antreiben des Mischelements 7 magnetisch mit dem Mischelement 7 gekoppelt ist. Hier ist also im Unterschied zum ersten Ausführungsbeispiel das Mischelement 7 nicht mehr der Rotor des elektrischen Drehantriebs, sondern der gesamte elektrische Drehantrieb inklusive seinem Rotor ist in der Antriebseinheit 8 vorgesehen.

Fig. 7 zeigt einen Schnitt durch die Antriebseinheit 8 und das Mischelement 7 entlang der Schnittlinie VII-VII in Fig. 8. Die Darstellung ist analog zur Darstellung in Fig. 2

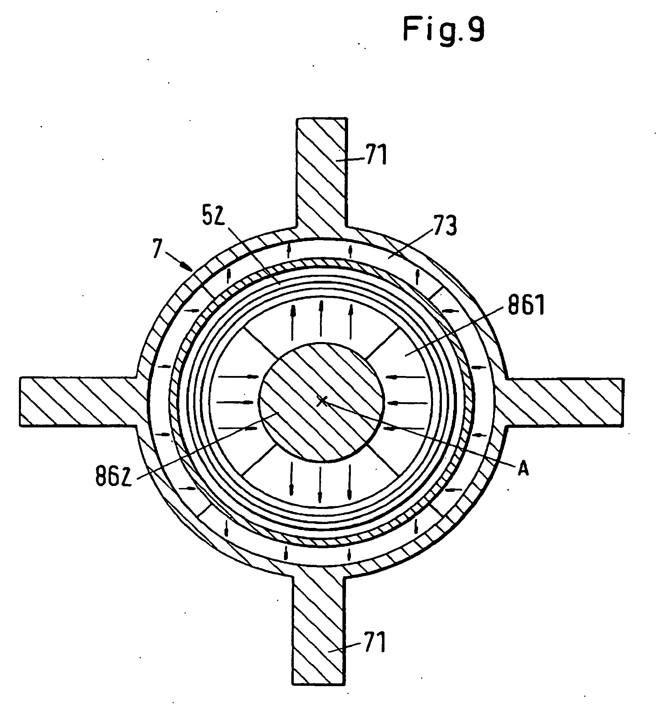

Fig. 8 und Fig. 9 zeigen jeweils die Antriebseinheit 8 und das Mischelement 7 in einer zu Fig. 7 senkrechten Schnittdarstellung entlang der Schnittlinie VIII-VIII bzw. IX-IX in Fig. 7.

Fig. 7 zeigt die Antriebseinheit 8 und das Mischelement 7 in einer Schnittdarstellung entlang der Längsrichtung A. Die Antriebseinheit 8, die in der Ausnehmung 5 angeordnet ist, umfasst einen elektrischen Drehantrieb 87, der einen Rotor 86 antreibt. Sowohl der elektrische Drehantrieb 87 als auch der Rotor 86 sind im Gehäuse 84 angeordnet, wobei - wie beim ersten Ausführungsbeispiel - eine gekapselte Ausführung möglich ist. Der Rotor 86 ist magnetisch mit dem Mischelement 7 gekoppelt, um dessen Rotation anzutreiben. Dazu umfasst der Rotor 86 (siehe insbesondere Fig. 9) einen Rotormagnet 861, der hier als ringförmiger Permanentmagnet ausgestaltet ist, welcher einen Rotorkern 862 umschliesst.

Der ringförmige Rotormagnet 861 ist bei diesem Ausführungsbeispiel als vierpoliger Magnet ausgestaltet (Polparzahl 2) und umfasst vier Ringsegmente, die jeweils homogen in radialer Richtung magnetisiert sind, wobei benachbarte Ringsegmente jeweils in die entgegengesetzte Richtung magnetiesiert sind, wie dies durch die Pfeile ohne Bezugszeichen angedeutet ist. Hieraus resultiert ein vierpoliger blockförmig oder rechteckförmig magnetisierter Rotor 861. Selbstverständlich sind noch viele andere Rotormagnetisierungen möglich.

Wie dies insbesondere in Fig. 7 zu erkennen ist, weist die becherartige Verstärkung 52 an ihrem darstellungsgemäss unteren Ende einen zentral angeordneten Stift 54 auf, auf dem ein konisches Führungselement 53 vorgesehen ist, welches mit einem ebenfalls konisch ausgestalteten Lagerelement 76 im Mischelement 7 zusammenwirkt, um so das Mischelement 7 bezüglich der Ausnehmung 5 zu zentrieren bzw. zu führen.

Das Mischelement 7 mit dem Grundkörper 72 weist die sich radial nach aussen erstreckenden Flügel 71 aus. In dem Grundkörper 72 ist der ringförmige Permanentmagnet 73 vorgesehen, der bei diesem Ausführungsbeispiel als vierpoliger Magnet ausgestaltet (Polparzahl 2) und vier Ringsegmente umfasst, die jeweils homogen in radialer Richtung magnetisiert sind, wobei benachbarte Ringsegmente jeweils in die entgegengesetzte Richtung magnetiesiert sind, wie dies durch die Pfeile ohne Bezugszeichen in Fig. 9 angedeutet ist. Hieraus resultiert ein vierpoliges blockförmig oder rechteckförmig magnetisiertes Mischelement 7.

Im Betriebszustand wird der Rotor 86 durch den Drehantrieb 87 in Rotation versetzt. Über die magnetische Kopplung wird dann auch das Mischelement 7 in Drehung versetzt, wobei es durch das Zusammenspiel des Lagerelements 76 mit dem Führungselement 53 bezüglich der Ausnehmung 5 zentriert und geführt wird. Wenn nun der Drehantrieb 87 mit dem Rotor 86 in der Ausnehmung 5 entlang der Längsrichtung A bewegt wird, also gemäss der Darstellung in Fig. 7 auf und ab bewegt wird, so folgt das Mischelement 7 dieser Bewegung. Bei einer darstellungsgemässen Aufwärtsbewegung wird dabei die Wandung 51 zusammengeschoben, bei einer darstellungsgemässen Abwärtsbewegung dehnt sich die Wandung 51 entlang der Längsrichtung A aus.

Bezüglich der Bewegung der Antriebseinheit 8 und des Mischelements 7 gelten die Ausführungen, die für das erste Ausführungsbeispiel gemacht wurden, in sinngemäss gleicher Weise auch für das zweite Ausführungsbeispiel. Insbesondere ist es auch bei dem zweiten Ausführungsbeispiel eine mögliche Variante, dass die Antriebseinheit 8 und damit das Mischelement 7 mindestens in einer Richtung quer zur Längsrichtung A bewegbar bzw. positionierbar ist. Es versteht sich, dass auch das zweite Ausführungsbeispiel so ausgebildet sein kann, dass der Rotor 86, welcher die Drehbewegung des Mischelements 7 antreibt, aussenliegend bezüglich des magnetisch wirksamen Teils des Mischelements 7 angeordnet sein kann - beispielsweise in sinngemäss gleicher Weise, wie dies in Fig. 5 dargestellt ist.

Natürlich sind für die Lagerung des Mischelements 7 bezüglich der Ausnehmumg 5 bzw. der Antriebseinheit 8 auch andere Varianten möglich, beispielsweise separate Magnetlager.

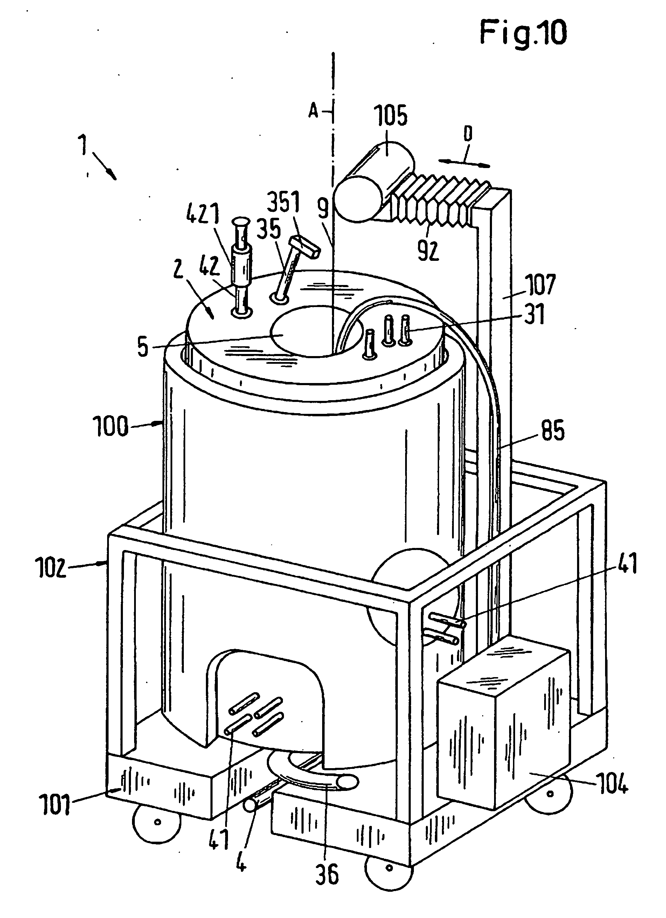

In Fig. 10 ist in einer schematischen Darstellung ein drittes Ausführungsbeispiel einer erfindungsgemässen Mischvorrichtung 1 dargestellt. Dieses dritte Ausführungsbeispiel lässt sich insbesondere unter Verwendung des ersten Ausführungsbeispiels oder unter Verwendung des zweiten Ausführungsbeispiels realisieren. Die Bezugszeichen haben die bereits erläuterten Bedeutungen.

Bei dieser Mischvorrichtung 1, die insbesondere ein Bioreaktor oder ein Fermenter sein kann, ist ein starres Stützgefäss 100 vorgesehen, welches aus Chromstahl, Edelstahl oder Kunststoff gefertigt ist. Dieses starre Stützgefäss 100 nimmt den Behälter 2 auf, der besonders bevorzugt als flexibler Beutel beispielsweise als Plastiksack ausgestaltet ist und inbesondere als Wegwerf-Behälter für den Einmalgebrauch. Die in dieser Darstellung nicht sichtbare Antriebsvorichtung 8 sowie das Mischelement 7 und die Einrichtungen zur Bewegung der Antriebsvorrichtung 8 können wie im ersten und/oder zweiten Ausführungsbeispiel ausgestaltet sein. Das starre Stützgefäss 100 ist auf einem fahrbaren Gestell 101 angeordnet und wird durch ein Geländer 102 gegen Verrutschen bzw. Stürzen gesichert.

Auf dem Gestell 101 ist ferner ein Ständer 107 montiert, an dessen darstellungsgemäss oberen Ende das eine Ende der Stange 92 vorgesehen ist. Am anderen Ende der Stange 92 ist die Wickelvorrichtung 105 montiert, mit welcher die Antriebseinheit 8 und damit das Mischelement 7 in Richtung der Längsrichtung A auf und ab bewegbar ist. Wie bereits weiter vorne erläutert, kann die Stange 92 so ausgestaltet sein, dass mit ihr die Wickelvorrichtung 105 und damit die Antriebseinheit 8 quer zur Längsrichtung A hin und her bewegbar ist, wie dies der Doppelpfeil D in Fig. 10 andeutet. Auch die bereits erläuterte Dreh- bzw. Schwenkbewegung der Stange 92 kann realisiert sein.

An dem Gestell 101 und/oder an dem starren Stützgefäss 100 ist ferner die Steuer- und Versorgungseinheit 104 vorgesehen, welche zum einen über die Versorgungsleitungen 85 die Antriebseinheit versorgt, steuert und kontrolliert und zum anderen die Bewegung der Antriebseinheit 8 relativ zu der Ausnehmung 5 steuert, also insbesondere die Wickelvorrichtung 105 und gegebenenfalls die Bewegung der Stange 92.

In dem Stützgefäss können weitere Einrichtungen vorgesehen sein, beispielsweise Einrichtungen zum Temperieren des Behälters 2 bzw. seines Inhalts. Auch diese weiteren Einrichtungen können von der Steuer- und Versorgungseinheit 104 versorgt und/oder angesteuert werden.

Dieses dritte Ausführungsbeispiel ermöglicht eine optimale Nutzung von flexiblen Behältern 2, wie beispielsweise Plastiksäcken. Nach dem jeweiligen Gebrauch kann der Behälter 2 entsorgt werden und durch einen neuen Wegwerf-Behälter 2 ersetzt werden. Dadurch entfallen beispielsweise aufwändige Sterilisierungsarbeiten.

Durch die Erfindung wird ferner ein Behälter 2 für eine Mischvorrichtung vorgeschlagen, der insbesondere für eine errfindungsgemässe Mischvorrichtung 1 geeignet ist. Der Behälter 2 ist als flexibler Beutel für den Einmalgebrauch ausgestaltet und enthält ein permanentmagnetisches Mischelement 7, das wie in den vorangehend beschriebenen Ausführugsbeispielen ausgestaltet sein kann. Der Behälter 2 weist eine zentrale Ausnehmung 5 auf, in welcher eine Antriebseinheit 8 bewegbar angeordnet werden kann, wobei diese Bewegung über einen wesentlichen Teil und vorzugsweise über die gesamte Erstreckung des Behälters 2 in Richtung der Ausnehmung 5 möglich ist. Vorzugsweise wird die Ausnehmung 5 durch eine Wandung 51 begrenzt, die flexibel ist und so ausgestaltet ist, dass die Ausdehnung der Ausnehmung 5 in ihrer Längsrichtung A veräbderbar ist.

Der Behälter 2 kann als flexibler Beutel oder als sterilisierter flexibler Beutel ausgestaltet und/oder verpackt sein.

## Patentansprüche

1. Mischvorrichtung mit einem Behälter (2) zum Mischen von Medien, welcher mindestens einen Einlass (31,32,33,34,35,36) für die zu mischenden Medien oder mindestens einen Auslass (4) aufweist, wobei eine zentrale Ausnehmung (5) vorgesehen ist, die sich in einer Längsrichtung (A) in den Behälter hinein erstreckt und durch eine Wandung (51) gegen den Innenraum (6) des Behälters (2) abgegrenzt ist, wobei in dem Behälter (2) benachbart zu der Ausnehmung (5) ein permanentmagnetisches Mischelement (7) zum Mischen der Medien vorgesehen ist und wobei in der Ausnehmung (5) eine Antriebseinheit (8) vorgesehen ist, mit welcher das Mischelement (7) über eine magnetische Kopplung in Rotation versetzt werden kann, wobei die Antriebseinheit (8) in der Ausnehmung (5) bewegbar angeordnet ist, sodass die Position des Mischelements (7) in dem Behälter kontrolliert veränderbar ist, **dadurch gekennzeichnet, dass** die Wandung (51) der Ausnehmung (5) flexibel und derart ausgestaltet ist, dass die Ausdehnung der Ausnehmung (5) in der Längsrichtung (A) veränderbar ist

2. Mischvorrichtung nach Anspruch 1, bei welcher die Antriebseinheit (8) ein elektrischer Drehantrieb (87) ist, der zum Antreiben des Mischelements (7) magnetisch mit dem Mischelement (7) gekoppelt ist.

3. Mischvorrichtung nach Anspruch 1, bei welcher die Antriebseinheit (8) einen Stator (80) umfasst, welcher zusammen mit dem Mischelement (7) als Rotor einen elektrischen Drehantrieb bildet.

4. Mischvorrichtung nach Anspruch 3, bei welcher das Mischelement (7) als permanentmagnetischer Rotor ausgestaltet ist, der zusammen mit dem Stator (80) einen lagerlosen Motor bildet, bei welchem der Stator (80) als Lager- und Antriebsstator für den permanentmagnetischen Rotor ausgestaltet ist.

5. Mischvorrichtung nach einem der vorangehenden Ansprüche, bei welcher die Antriebseinheit (8) in der Ausnehmung (5) hängend angeordnet ist.

6. Mischvorrichtung nach einem der vorangehenden Ansprüche, bei weicher das innenliegende Ende der Ausnehmung (5) eine becherartige Verstärkung (52), insbesondere aus Kunststoff aufweist.

7. Mischvorrichtung nach eine der vorangehenden Ansprüche, bei welchem die Antriebseinheit (8) quer zur Längsrichtung (A) der zentralen Ausnehmung (5) bewegbar ist.

8. Mischvorrichtung nach einem der vorangehenden Ansprüche, bei welchem der Behälter (2) als flexibler Beutel oder als sterilisierter flexibler Beutel ausgestaltet ist.

9. Mischvorrichtung nach einem der vorangehenden Ansprüche, bei welchem der Behälter (2) als Wegwerf-Behälter für den Einmalgebrauch ausgestaltet ist.

10. Mischvorrichtung nach einem der vorangehenden Ansprüche mit einem starren Stützgefäss (100), welches den Behälter (2) aufnimmt, der vorzugsweise als flexibler Beutel ausgestaltet ist.

11. Mischvorrichtung nach einem der vorangehenden Ansprüche, welches als Bioreaktor oder als Fermenter ausgestaltet ist.

12. Behälter für eine Mischvorrichtung, die gemäss einem der vorangehenden Ansprüche ausgestaltet ist, wobei der Behälter (2) als flexibler Beutel für den Einmalgebrauch ausgestaltet ist, ein permanentmagnetisches Mischelement (7) enthält und eine zentrale Ausnehmung (5) aufweist, in welcher eine Antriebseinheit (8) bewegbar angeordnet werden kann, **dadurch gekennzeichnet, dass** die Ausnehmung (5) durch eine Wandung (51) begrenzt wird, die flexibel ist und so ausgestaltet ist, dass die Ausdehnung der Ausnehmung (5) in einer Längsrichtung (A) veränderbar ist.

## Claims

1. A mixing apparatus having a container (2) for the mixing of media which has at least one inlet (31, 32, 33, 34, 35, 36) for the media to be mixed or at least one outlet (4), with a central cut-out (5) being provided which extends in a longitudinal direction (A) into the container and is bounded relative to the inner space (6) of the container (2) by a wall (51), with a permanent magnetic mixing member (7) being provided in the container (2) adjacent to the cut-out (5) for the mixing of the media, and with a drive unit (8) being provided in the cut-out (5) by which the mixing member (7) can be set into rotation via a magnetic coupling, with the drive unit (8) being movably arranged in the cut-out (5) such that the position of the mixing member (7) in the container is variable in a controlled manner, **characterized in that** the wall (51) of the cut-out (5) is designed to be flexible and such that the extent of the cut-out (5) is variable in the longitudinal direction (A).

2. A mixing apparatus in accordance with claim 1, wherein the drive unit (8) is an electrical drive unit (87) which is magnetically coupled with the mixing member (7) to drive the mixing member (7).

3. A mixing apparatus in accordance with claim 1, wherein the drive unit (8) includes a stator (80) which, together with the mixing member (7) as a rotor, forms an electrical drive unit.

4. A mixing apparatus in accordance with claim 3, wherein the mixing member (7) is designed as a permanent magnetic rotor which, together with the stator (80), forms a bearing-free motor in which the stator (80) is designed as a bearing and drive stator for the permanent magnetic rotor.

5. A mixing apparatus in accordance with any one of the preceding claims, wherein the drive unit (8) is arranged suspended in the cut-out (5).

6. A mixing apparatus in accordance with any one of the preceding claims, wherein the inwardly disposed end of the cut-out (5) has beaker-like reinforcement (52), in particular made of plastic.

7. A mixing apparatus in accordance with any one of the preceding claims, wherein the drive unit (8) is movable transversely to the longitudinal direction (A) of the central cut-out (5).

8. A mixing apparatus in accordance with any one of the preceding claims, wherein the container (2) is designed as a flexible bag or as a sterilized flexible bag.

9. A mixing apparatus in accordance with any one of the preceding claims, wherein the container (2) is designed as a disposable container for single use only.

10. A mixing apparatus in accordance with any one of the preceding claims having a rigid support vessel (100) which receives the container (2) which is preferably designed as a flexible bag.

11. A mixing apparatus in accordance with any one of the preceding claims, which is designed as a bioreactor or as a fermenter.

12. A container for a mixing apparatus which is designed in accordance with any one of the preceding claims, wherein the container (2) is designed as a flexible bag for disposable use, includes a permanent magnetic mixing member (7) and has a central cut-out (5) in which a drive unit (8) can be movably arranged, **characterized in that** the cut-out (5) is bounded by a wall (51) which is flexible and is designed such that the extent of the cut-out (5) is variable in a longitudinal direction (A).

## Revendications

1. Dispositif de mélange avec un contenant (2) pour le mélange de milieux, qui comprend au moins une entrée (31, 32, 33, 34, 35, 36) pour les milieux à mélanger ou au moins une sortie (4), où est prévu un évidement central (5) qui s'étend dans une direction longitudinale (A) dans le contenant et qui est délimité par une paroi (51) relativement à l'espace intérieur (6) du contenant (2), où est prévu dans le contenant (2), d'une manière avoisinante à l'évidement (5), un élément de mélange (7) à aimant permanent pour le mélange des milieux, et où est prévu dans l'évidement (5) une unité d'entraînement (8) au moyen de laquelle l'élément de mélange (7) peut être entraîné en rotation par un couplage magnétique, où l'unité d'entraînement (8) est disposée d'une manière mobile dans l'évidement (5) de sorte que la position de l'élément de mélange (7) dans le contenant peut être modifiée d'une manière contrôlée, **caractérisé en ce que** la paroi (51) de l'évidement (5) est réalisée d'une manière flexible et de telle sorte que l'extension de l'évidement (5) dans la direction longitudinale (A) est modifiable.

2. Dispositif de mélange selon la revendication 1, dans lequel l'unité d'entraînement (8) est un entraînement en rotation électrique (87) qui, pour l'entraînement de l'élément de mélange (7), est couplé magnétiquement à l'élément de mélange (7).

3. Dispositif de mélange selon la revendication 1, dans lequel l'unité d'entraînement (8) comprend un stator (80) qui, conjointement avec l'élément de mélange (7) comme rotor, forme un entraînement en rotation électrique.

4. Dispositif de mélange selon la revendication 3, dans lequel l'élément de mélange (7) est réalisé comme rotor à aimant permanent qui, conjointement avec le stator (80), forme un moteur sans palier dans lequel le stator (80) est réalisé comme stator de palier et d'entraînement pour le rotor à aimant permanent.

5. Dispositif de mélange selon l'une des revendications précédentes, dans lequel l'unité d'entraînement (8) est disposée de façon à pendre dans l'évidement (5).

6. Dispositif de mélange selon l'une des revendications précédentes, dans lequel l'extrémité située à l'intérieur de l'évidement (5) présente un renforcement en forme de pot (52), en particulier en matériau synthétique.

7. Dispositif de mélange selon l'une des revendications précédentes, dans lequel l'unité d'entraînement (8) est déplaçable transversalement à la direction longitudinale (A) de l'évidement central (5).

8. Dispositif de mélange selon l'une des revendications précédentes, dans lequel le contenant (2) est réalisé comme sac flexible ou comme sac flexible stérilisé.

9. Dispositif de mélange selon l'une des revendications précédentes, dans lequel le contenant (1) est réalisé comme contenant jetable pour un usage unique.

10. Dispositif de mesure selon l'une des revendications précédentes avec un récipient de support rigide (100) qui reçoit le contenant (2) qui est réalisé de préférence comme sac flexible.

11. Dispositif de mélange selon l'une des revendications précédentes, qui est réalisé comme bioréacteur ou fermenteur.

12. Contenant pour un dispositif de mélange qui est réalisé selon l'une des revendications précédentes, où le contenant (2) est réalisé comme sac flexible pour l'usage unique, contient un élément de mélange (7) à aimant permanent et présente un évidement central (5) dans lequel une unité d'entraînement (8) peut être disposée d'une manière mobile, **caractérisé en ce que** l'évidement (5) est délimité par une paroi (51) qui est flexible et qui est réalisée de façon que l'extension de l'évidement (5) dans une direction longitudinale (A) est modifiable.
